# EUROPEAN PATENT APPLICATION

(11) **EP 0 719 792 A2**
(43) Date of publication of application: **03.07.1996**
(21) Application number: 96101997.3
(22) Date of filing: 23.01.1990
(51) Int. Cl.: C07K 14/61, C12N 15/18

(54) **Somatotropin variants**

(30) Priority: 15.03.1989 US 323901
(62) Divisional of application: 90906035.2
(71) Applicant: THE UPJOHN COMPANY, Kalamazoo, Michigan 49001 (US)
(72) Inventor: Brunner, David Paul, Portage, Michigan 49081 (US); Garlick, Robert Lee, Augusta, Michigan 49012 (US); Harbour, Gary Clyde, Kalamazoo, Michigan 49009 (US); Lyle, Stephen Baker, Kalamazoo, Michigan 49001 (US); Mott, John Edward, Kalamazoo, Michigan 49004 (US); Shafer, Jules A., Ann Arbor, Michigan 48104 (US)
(74) Representative: Perry, Robert Edward

(57) **Abstract**

An animal somatotropin which has at least one lysine residue corresponding to residues 157, 167, 171 or 180 of the native somatotropin sequence replaced by arginine or glutamine.

## Description

### FIELD OF INVENTION

This invention is in the fields of microbiology and protein chemistry. More particularly, this invention relates to fermentation processes for producing recombinant heterologous polypeptides, particularly bovine somatotropin (rbSt) and a novel rbSt produced thereby.

### BACKGROUND OF THE INVENTION

Synthetic and chemically defined media for cultivating microorganisms are well known. Conventional nutrient media for cultivating bacteria have been used to grow recombinant bacteria that produce heterologous polypeptides. Decreasing the amount of expensive amino acids in growth media, while at the same time keeping expression of good quality heterologous polypeptides at a high level, is desirable. During fermentations in such media, production of novel acetylated analogs of heterologous polypeptides, for example rbSt, can be accomplished.

Naturally occurring bovine somatotropin (bSt), or growth hormone, can be purified from the pituitary glands of cattle as a mixture of heterogeneous proteins (Paladini, A. C. et al., 1983. CRC Reviews in Biochem., 15:25-56). Undefined electrophoretic heterogeneity is seen when native bovine pituitary extracts are fractionated by anion exchange chromatography (Hart, I.C. et al., 1984. Biochem. J., 218:573-581). Pituitary bovine somatotropin (pbSt) exists in isoforms which differ in isoelectric point (pI), with the most abundant form of pI 8.2. bSt is known to possess sites that can undergo deamidation, resulting in an increase in the number of lower pI forms which are seen as clusters of closely-spaced bands on isoelectric focusing at pI 7.0, 6.0, and 5.5 (Lewis, U.J., et al., 1970. Biochim. Biophys. Acta., 214:498-508; Secchi, C., et al., 1986. Int. J. Peptide Res. 28:298-306).

### INFORMATION DISCLOSURE

Acetylation of bovine somatotropin, principally at the ε-amino groups of lysine residues, results in a reduction in the positive charge of the protein due to the neutral nature of the acetyl moiety, thus producing bSt species with reduced pIs. Methods for chemical acetylation of bSt have been reported, but such means result in many acetylated products because of reactions with several other amino acids in addition to lysine (Oikawa, A., et al., 1967. Biochem. J., 104:947-952; De Satz, V.B. and J.A. Santome', 1981. Int. J. Peptide Protein Res. 18:492-499).

The acetylation of naturally occurring proteins is known. A dipeptide derived from Tobacco Mosaic virus was first shown to be modified by acetylation (Narita, K., 1958. Biochim. Biophys. Acta, 28:184-191). Subsequently, a large number of proteins, including ovalbumin, egg albumin, hemoglobins, and histones, have been shown to contain acetylated amino acids (Harris, J.I., 1959. Biochem. J. 71:445-459; Narita, K., 1961. Biochem. Biophys. Res. Comm. 5:160-164; Schroeder, W. A. et al., 1962. Biochim. Biophys. Acta, 63:532-534; Phillips, D.M.P., 1963. Biochem. J., 87:258-263). N^{α}-acetylation of proteins is widespread among eukaryotic and prokaryotic organisms and viruses (Driessen, H.P.C. et al., 1985. CRC Crit. Rev. Biochem., 18:281-325). For example, it has been suggested that 80% of the soluble proteins of Ehrlich ascites cells are N^{α}-acetylated (Brown, J.L. and W.K. Roberts, 1976. J. Biol. Chem., 251:1009-1014). N^{α}-acetylation appears to be a non-random process, as alanine and serine residues are the prime targets, whereas methionine, glycine, and aspartic acid are modified in only a few proteins, and several amino acids are not known to be N^{α}-acetylated (Wold, F., 1981. Ann. Rev. Biochem., 50:783-814).

Protein side-chain acetylation is another class of protein acetylation known to occur naturally, albeit less frequently than N^{α}-acetylations. The N^{ε}-acetylases are distinct from the N^{α}-acetylases, as they are specific for the ε-amino group of lysine and are located in the nucleus or cytosol, while the N^{α}-acetylases appear to be ribosome-associated (Sterner, R. et al., 1979. J. Biol. Chem., 254:11577-11583; Pestana, A. and H. C. Pitot, 1975. Biochemistry, 14:1397-1403; Pestana, A. and H.C. Pitot, 1975. Biochemistry, 14:1404-1412). However, both types of acetylases catalyze the transfer of acetyl from acetyl CoA to proteins (Wold, F., supra).

N^{ε}-acetylation of lysine residues was first shown to occur with histones H3 and H4 (Allfrey, V.G., et al., 1964. Proc. Nat. Acad. Sci. U.S.A., 51:786-794). In addition to histones, other DNA-binding proteins such as high-mobility group (HMG) proteins are N^{ε}-acetylated (Sterner, R., et al., supra).

Few proteins obtained from *Escherlchia coli* strains have been reported to be acetylated. The *E. coli* pyruvate dehydrogenase complex is acetylated at lipoyl residues which are linked to N^{ε}-amino groups of lysine resides on the protein (Adamson, S.R., et al., 1986. Biochem. Cell Biol., 64:250-255). Most reports concerning protein acetylation in *E. coli* have concentrated on acetylation of ribosomal proteins. N^{α}-acetylations of serine residues on *E. coli* ribosomal protein L7 (to produce protein L12) and alanine residues on ribosomal proteins S5 and S18 have been described (Brot, N. and H. Weissbach, 1972. Biochem. Biophys. Res. Commun. 49:673-679; Wittmann-Liebold, B. and B. Greurer, 1978. FEBS Lett., 95:91-98; Yaguchi, M., 1976. FEBS Lett. 59:217-220).

There are no previous reports of acetylated heterologous polypeptides produced by host microorganisms, e.g., *E. coli*, and there are no previous specific reports of acetylated bSt.

### SUMMARY OF THE INVENTION

The present invention relates generally to fermentations for the production of acetylated heterologous polypeptides, particularly analogs of recombinantly-produced bovine somatotropin (rbSt) and the novel rbSt produced thereby.

More specifically, the present invention relates to a method to produce an acetylated analog of rbSt by growing microorganisms on a defined fermentation medium having a low concentration of amino acids.

More specifically, the present invention relates to a novel acetylated analog of rbSt.

More specifically, the microorganism is a recombinant microorganism and the rbSt so expressed is a heterologous polypeptide.

The present invention also relates to methods for preventing production of acetylated rbSt by replacement of specific lysine residues with arginine and/or glutamine residues and the novel rbSt molecules so produced.

### DETAILED DESCRIPTION OF THE INVENTION

As used herein, "heterologous polypeptide(s)" refers to polypeptides not naturally synthesized by a transformed host microorganism of interest. For example, an *E. coli* host may produce bSt, insulin, interferon, etc. The polypeptides so produced are called "heterologous polypeptides." Of particular interest in the context of this invention are those heterologous polypeptides comprising mammalian somatotropins, e.g., bSt.

As used herein, "a low concentration of amino acids" means a concentration that is low enough such that it can be reduced by a microorganism cultured thereon to a "minimal concentration of amino acids", which is in turn defined as the concentration of exogenous amino acids which is too low to repress the biosynthesis of, or feedback inhibit effectively the activities of, amino acid biosynthetic enzymes. Such a reduction of the "low concentration of amino acids" to a "minimal concentration of amino acids" occurs during or slightly preceding the biosynthesis of the heterologous polypeptide. For example, with *E. coli* hosts and the rbSt polypeptide of the specific examples, the "low concentration of amino acids" is less than about 0.50 mM each, as derived from a 0.1% yeast extract supplement. The "low concentration of amino acids" is reduced to a "minimum concentration of amino acids" which is less than about 0.05 mM each.

Recombinant host microorganisms used in this invention are made by recombinant DNA techniques well known to those skilled in the art and set forth, for example, in *Molecular Cloning*, T. Maniatis, et al., Cold Spring Harbor Laboratory (1982) and *A Practical Guide to Molecular Cloning*, Perbal, B., John Wiley & Sons (1984), which are incorporated herein by reference. Useful host microorganisms include any of the well known hosts suitable for cloning and expressing heterologous genes and including, for example, *Bacillus*, *Saccharomyces* and *Streptomyces* strains.

The heterologous polypeptide-producing recombinant microorganisms are cultivated in liquid nutrient medium. The medium comprises an excess of conventional nutrient materials that fulfill the cellular growth requirements of the microorganism, thereby enabling the microorganism to grow and multiply to a predetermined cellular density. This material includes sources of carbon, nitrogen and minerals such as sulfur, phosphorous, magnesium, potassium, copper, zinc, manganese, and iron. Amino-acids may be added in the form of protein hydrolysates usually made by subjecting naturally occurring proteinaceous materials, such as casein, soybean meal, lactalbumin, animal tissue, yeast cells and gelatin, to acid or enzymatic digestion. Mixtures of pure amino-acids may also be added.

Oxygen is also provided to the medium. To achieve maximum culture densities, the cultivation will usually be done in a way to enhance the area of the oxygen/liquid interface.

Important environmental factors affecting the cultivation include pH and temperature. The temperature will range between the minimum and maximum growth temperatures. Most bacteria exhibit maximum growth over a fairly narrow temperature range. For mesophilic bacteria, such as *E. coli*, the optimum temperature range is about 25°C to about 42°C, preferably about 37°C. Most organisms will tolerate hydrogen ion concentrations ranging over several pH units. For bacteria, such as *E. coli*, the tolerable pH lies in the range of about 6 to 8, with about 6.8 being preferred.

If expression of a gene encoding a heterologous polypeptide is under control of a repressible expression control sequence, one can specifically repress expression of that gene until a predetermined level of growth is reached by the cell culture by adding an appropriate repressor to the medium (e.g., tryptophan when expression is under control of the tryptophan promoter and operator).

After harvest, the cells are processed to recover the heterologous polypeptide. This normally involves disrupting the cells, separating crude heterologous polypeptide from bacterial proteins by one or more extraction steps, solubilizing the polypeptide (depending upon its hydrophobicity), oxidizing sulfhydryls to form proper disulfide bonds, when appropriate, and further purifying the polypeptide by ion-exchange chromatography, gel filtration, high performance liquid chromatography or other protein purification procedures.

We have been running fermentations to produce heterologous polypeptides by recombinant microorganisms, for example, rbSt in *E. coli* K-12 strains. These fermentations involve "low yeast extract" fermentation media containing 0.1% yeast extract, and effectively support over-expression of polypeptides in recombinant microorganisms.

Due to the molecular heterogeneity of somatotropins, the position numbers of amino acid residues of the various somatotropins may differ. The term "native mammalian somatotropin" includes these naturally occurring species. Chart 1 illustrates the specific amino acid sequence of one species of bSt. The numbering for other somatotropins may differ where analogs are involved. Those of ordinary skill in the art can readily locate corresponding amino acid sequences in alternative native mammalian somatotropins or their analogs.

pbSt exists in isoforms which differ in their respective isoelectric points. The most abundant form exhibits a pI of 8.2, with clusters of closely-spaced bands being evident on isoelectric focusing at pI 7.0, 6.0, and 5.5. RbSt was also found to contain lower pI species in clusters with pI of 7.0, 6.0, and 5.5. Incubation of rbSt in aqueous base such as 0.1M NH₄CO₃ (pH 10), causes a major shift to species with lower isoelectric points. Extensive structural analyses showed the modification following base treatment involved a conversion of the asparagine located at amino acid residue 99 (chart 1) to isoaspartic and aspartic acids (see copending U.S. patent application S.N. 07/299,107, filed 19 February 1989, which is incorporated herein by reference).

Isolated rbSt which contained a replacement of the asparagine located at amino acid residue 99 (Asn99) (chart 1) with serine (Ser99), thus avoiding formation of isoaspartic acid, also contained rbSt species with pI of 7.0, 6.0, and 5.0. The pI 7.0 species of Ser99 rbSt accounted for approximately 30% of the total rbSt obtained. Independent structural analyses using purified pI 7.0 material of native rbSt (Asn99) showed at least four of the pI 7.0 species were formed by acetylation of lysine residues 157, 167, 171, and 180 (chart 1). Without such single acetylations, a native pI of 8.2 would be observed for Ser99 rbSt. Simultaneous acetylation of multiple lysine residues results in rbSt species exhibiting pI <7.0. The acetylation of other rbSt lysine residues (positions 30, 64, 70, 112, 114, 139, and 144, chart 1) has not been detected but may exist.

The purposeful acetylation of lysine residues during fermentations for protein overexpression makes available the synthesis of proteins with greater resistance to proteolytic degradation by trypsin and trypsin-like proteases and lysine-specific modifying reagents. Furthermore, as acetylation of lysine residues on the ε-amino group results in a reduction of the polypeptide's pI by one unit per acetylation, such polypeptides would be expected to be less susceptible to clearing by the kidneys from the circulation of a mammal administered the polypeptide. A lower pI would also be expected to increase the solubility of rbSt under the conditions used for isolation and formulation.

On the other hand, it may be desirable to produce rbSt without acetylation at lysine residues. This would allow production of homogeneous products with attendant savings in product purification.

The acetylation of specific lysine residues during fermentations for the overexpression of rbSt was unexpected, and the mechanisms of such acetylation are not known. Prior to this invention there were no known fermentation means for obtaining rbSt with acetylations at lysine residues. Furthermore, there were no known means for preventing the acetylation of specific lysine residues during overexpression of rbSt.

Bacterial cultures: *E. coli* strain DU45 was used. This strain was derived from a wild-type strain of *E. coli* K-12 (ATCC e23716) by removal of the lambda prophage and the F plasmid and by introduction of the *rpoH112* allele This strain was then transformed with plasmid pURA-99Ser-M4, a temperature-sensitive runaway replication vector derived from plasmid pURA-4 and containing a gene encoding bSt (PCT patent application PCT/US 88/00328, incorporated herein by reference) having the asparagine at residue 99 (Chart 1) replaced with serine (see copending U.S. application S.N. 07/299,107).

Culture Media: The defined media were based upon medium E of Vogel and Bonner (Vogel, H. J. and D. M. Bonner, 1955. J. Biol. Chem., 218:97-106). The compositions and protocols for preparing the primary-seed and fermentation media are as follows:

### Primary-Seed Medium

| Components | Amount per Liter |
|---|---|
| Na(NH₄)HPO₄·H₂O | 10.90 g |
| K₂HPO₄ | 2.61 g |
| Citric Acid·H₂O | 2.10 g |
| MgSO₄·7H₂O | 0.99 g |
| (NH₄)₂SO₄ | 0.66 g |
| Yeast Extract | 10.40 g |
| Glycerol | 5.00 g |
| R.O. Water | |

For primary-seed cultures, the above components were hydrated with water (reverse-osmosis grade, R.O.) by q.s. to 1000 ml, and divided into 300-ml volumes prior to sterilization. Seed medium was sterilized for 20 minutes at 121°C. Following sterilization, the seed medium was allowed to cool, and 0.5 ml sterile ampicillin (25 g/liter in R.O. water titrated to pH 8.0 with NaOH and filter sterilized, 0.45 µm) was added.

### Fermentation Medium

| Components | Amount per Liter |
|---|---|
| Na(NH₄)HPO₄·H₂O | 10.90 g |
| K₂HPO₄ | 2.61 g |
| Citric Acid (anhydrous) | 1.92 g |
| MgSO₄·7H₂O | 0.25 g |
| (NH₄)₂SO₄ | 0.66 g |
| Yeast Extract | 1.00 g |
| SAG4130 | 0.75 ml |
| R.O. Water | |

The fermentation medium was prepared by increasing the amounts of the above components 200-fold and hydrating them in 165 liters of R.O. water in a 250-liter fermentor. The fermentation medium was then sterilized for 20 minutes at 121°C (during sterilization, a medium volume increase of 20 liters was assumed, due to steam condensation). Following sterilization, the pH of the medium was checked to confirm a pH of 6.6 to 6.9. Upon cooling, two aseptic additions were made to complete the fermentation medium. The first addition consisted of a 200-ml aliquot of micronutrients (final concentrations: (NH₄)₆(MO₇)₂₄·4H₂O, 12 µM; H₃BO₃, 1.6 mM; Co-Cl₂·6H₂O, 120 µM; CuSO₄, 25.5 µM; MnCl₂·4H₂O, 319.4 µM; ZnSO₄·7H₂O, 40.1 µM) which had been sterilized by filtration (0.45 µm). The second addition consisted of 15 liters cerelose (8.24 kg cerelose q.s. to 14 liters with R.O. water and adjusted to pH 4.0 with H₂SO₄). The fermentation medium was adjusted to pH 7.2 with 25% NaOH prior to inoculation.

The low yeast extract fermentation medium contained 0.1% yeast extract for DU45 cultures.

### EXAMPLE 1 Production of Acetylated rbSt During Low Yeast Extract Fermentations

Each primary-seed culture was inoculated with the contents of one culture ampoule (about 1 ml) and incubated at 28°C to a culture density of 0.7 to 0.8 A₅₅₀. Following incubation, each primary-seed culture was placed on ice prior to use for inoculation of the fermentors. All primary-seed cultures were supplemented with ampicillin to yield cultures containing essentially all plasmid-bearing bacteria. DU45 fermentations were initiated by inoculation with a 600-ml inoculum. Fermentor pH was controlled with anhydrous ammonia to maintain the pH at 7.2 to 7.4. Fermentor agitation and aeration rates were set at 320 rpm and 300 slm, at a backpressure of 10 psig. Cultures were grown at a permissive temperature of 27°C. DU45 fermentations were maintained at 27°C throughout, because plasmid runaway replication and rbSt synthesis were spontaneously induced (PCT patent application PCT/US 88/003280).

Culture aliquots were removed at various times from the primary seeds and fermentor and subjected to a series of analytical procedures to quantitate the culture density (absorbance at 550 nm), total bacterial dry weight, total rbSt concentration, glucose concentration, free ammonia concentration, acetate concentration, and microscopic identification and quantitation of inclusion bodies.

Purified rbSt preparations were derived from fermentation cultures of strain DU45 and subjected to isoelectric focusing (IEF) to quantitate the levels of acetylated rbSt (pI 7.0 vs. 8.2 species of Ser99-rbSt). The greater the level of pI 7.0 rbSt, the greater the fraction of total rbSt in the monoacetylated form. IEF was performed in 1 mm thick horizontal slab gels of a 5% T, 3% C composition and with a pH range of 3.5 to 9.5 (LKB PAGplate 1804-101). Gels were run in the absence of any denaturant at 1500 V maximum and at a constant power of 30W at 10°C. Gels were fixed for 30 minutes at room temperature in aqueous 11.5% (w/v) trichloroacetic acid, 3.5% (w/v) sulfosalicylic acid. Staining was performed for 10 minutes at 60°C with 0.1% (w/v) Coomassie Blue R250 in 25% ethanol: 8% acetic acid. Gels were destained in 25% ethanol:8% acetic acid. Quantitation of rbSt species in IEF gels was done using a LKB laser densitometer.

Fermentation samples were obtained during the course of a low yeast extract fermentation and the levels of monoacetylated rbSt (pI 7.0 form of Ser99 rbSt) quantitated by IEF. The data in Table 1 show that the level of monoacetylated rbSt species in rbSt preparations ranges from a fractional percent of 24.9% to 34.7% during the course of the fermentation. Furthermore, these data show that the level of monoacetylated rbSt species is significant in early fermentation samples (e.g., 17 hours post-inoculation) and remains relatively constant throughout the duration of the fermentation.

### EXAMPLE 2 Site-Directed Mutagenesis of the Lysine Codons at Positions 157, 167, 171 and 180 of a rbSt Gene Containing Serine Codon Substitutions at Positions 99, 181 and 189.

### 1. The construction of a rbSt gene containing codons for serine at positions 99, 181, and 189.

The rbSt gene used for the modification of the four terminal lysines is designated m4. The m4 gene construction has been previously described (PCT patent application PCT/US 88/00328). The m4 gene was further modified by inserting serine codons at positions 99, 181 and 189. The construction of the vector pTrp-bGH-m4-99Ser which contains an m4 rbSt gene with a serine substitution at position 99 is set forth in copending U.S. application S.N. 07/299,107. The construction of the vector pDH-bSt-m4-Ser181/9 which contains an m4 rbSt gene with serine substitutions at positions 181 and 189 is set forth in copending U.S. application S.N. 07/304,733, filed on 31 January 1989 and incorporated herein by reference.

A m4 rbSt gene containing serine codons at positions 99, 181, and 189 is constructed by digesting the pTrp-bGH-m4-99Ser and pDH-bSt-m4-Ser181/9 vectors individually with the restriction enzymes EcoRI and MstII (New England BioLabs, Beverly, MA.). Each of the restriction enzymes cut the vectors once and generates a large vector fragment and a smaller fragment of about 814 basepairs (bp). The 814 bp fragment was isolated from the pTrp-bGH-m4-99Ser digestion by using techniques previously described (PCT/US 88/00328). This fragment contains the sequence for the *E. coli* tryptophan (*trp*) promoter, the *trpL* ribosome binding site (rbs) and the coding sequence for the rbSt m4-99Ser gene up to codon 175. The MstII restriction sequence is located over codons 175, 176 and 177 in the rbSt gene. This fragment contains the m4 and 99Ser changes. The vector fragment from the pDH-bSt-m4-Ser181/9 was isolated. This fragment retains the rbSt coding sequence for the 181 and 189 serine codon modifications. The two isolated fragments were ligated as described previously (PCT/US 88/00328) and were used to transform competent cells of *E. coli* strain MC1000 (available in the Experiments with Gene Fusion Strain Kit, Cold Spring Harbor Laboratory, Cold Spring Harbor, New York). Vector DNA was isolated from transformed cells and analyzed by DNA sequencing to confirm the presence of the serine codons at positions 99, 181, and 189. One of the vectors was designated pDH-m4-TS (Triple Serine).

### 2. Cloning of a fragment from pDH-m4-TS into M13mp9

The site-directed mutagenesis methodology employed requires cloning the target sequences into the M13mp9 vector. M13mp9 was provided in a "Site Directed Mutagenesis Kit" (Boehringer Mannheim Biochemicals, Indianapolis, IN). The M13mp9 dsDNA and the pDH-m4-TS vector are digested with the restriction enzymes PstI and BamHI. Each restriction enzyme cuts M13mp9 and pDH-m4-TS once. The digestion of M13mp9 generates two fragments, one of which is not detectable on gels. The visible M13mp9 fragment (approximately 7,230 bp) was isolated. The digestion of the pDH-m4-TS generates two fragments which have the approximate sizes of 3200 and 310 bp. Both fragments were isolated. The smaller fragment contains the rbSt coding sequences corresponding to amino acids 91 through 191, and includes the lysine codons at positions 157, 167, 171, and 180. The 310 bp fragment and the M13mp9 fragment were ligated and used to transfect competent cells of BHM 71-18 (this strain was provided in the mutagenesis kit, supra). The methods used for the transfection, identification of plaques containing inserts and isolation of single-stranded DNA (ssDNA) have been described (Messing, J., 1983. Meth. Enzymol. 101:20-98). Plaque formation was performed in the presence of a β-galactosidase indicator, X-Gal, and IPTG (available from Sigma Chemical Co., St. Louis, MO) as described by Messing (supra). ssDNA was prepared and the presence of the 310 bps fragment was confirmed by DNA sequencing. The vector was designated M13mp9-181/9S.

### 3. Substitution of the lysine codons at positions 157, 167, 171 and 180 to arginine codons.

The mutagenesis protocol provided in the Boehringer "Site Directed Mutagenesis Kit" was used. The M13mp9 rev dsDNA was digested with the restriction enzymes EcoRI and HindIII. The protocol is briefly described as follows: approximately 1.25 µg of ssDNA of M13mp9-181/9S was hybridized to 0.5 µg of EcoRI/HindIII cut M13mp9 rev dsDNA in the presence of the hybridization buffer provided. The annealing is performed by heating the mixture to 100°C for 4 minutes followed by slow cooling to 65°C. The mixture (40 µl) is maintained at 65°C for 20 minutes and is placed on ice. This produces a dsDNA molecule containing a single-stranded gap over the target DNA sequence. 8 µl of the gapped mixture is added to 2 µl of kinase oligonucleotide containing the desired base changes with a concentration of at least 4 pmol. The oligonucleotide contains all 4 lysine-to-arginine changes and is designed to be complementary to the sequence in the single stranded gapped region. The sequence of the oligonucleotide with the arginine codon changes underlined is as follows:
5' GAA ACG ACG GGA ACG CAT AAC CCT CAG GTA CGT CTC CGT ACG ATG CAG GTC GCG CCG GAA GCA GGA GAG CAG ACC GTA GTT ACG GAG CAG CGC
The primer is annealed by incubating at 65°C for 5 minutes and slow cooling to room temperature. After priming, the remainder of the gapped ssDNA region is converted to dsDNA by filling in the presence of dNTP, T4 DNA ligase and Klenow for 45 minutes at room temperature. The reaction is stopped by the addition of EDTA and heating to 65°C for 10 minutes. The reaction mixture is use to transfect competent cells of BHM 71-18 *mutS*. The transfected cells are grown overnight in LB broth and a M13 phage stock is prepared from the supernatant. The M13 phage are then plated on MK30-3 and plaques are chosen and ssDNA is prepared. Clones with the desired mutations are identified by sequence analysis.

### 4. Cloning the rbSt fragment containing the lysine-to-arginine changes into the pURA expression vector.

Double stranded M13 DNA is prepared from the lysine-to-arginine M13 phage as described by Messing (supra). The DNA is digested with the restriction endonucleases PstI and BamHI. This generates 7,200 and 310 bp fragments. The 310 bp fragment was isolated and ligated with the PstI/BamHI vector fragment from pDH-m4-TS previously isolated in section 2. The two fragments were ligated and used to transform competent cells of MC1000. Vector DNA was prepared from individual transformants and characterized by DNA sequencing. A candidate with the desired changes (99ser, 157arg, 167arg, 171arg, 180arg, 181ser and 189ser) was designated pDH-m4-TS/QA.

To clone the modified gene into the pURA expression vector (PCT/US 88/00328), the vector DNA of pDH-m4-TS/QA was digested with EcoRI and HindIII to generate vector fragments of approximately 2,800 bp and 850 bp, containing the trp promoter, the trpL rbs, and rbSt coding sequences up to position 186. The HindIII restriction site sequence occurs over the codons for amino acids 186, 187 and 188. The pURA-BStm4-181/9 vector described in copending U.S. patent application S.N. 07/304,733, was digested with EcoRI and BamHI. This digestion produces vector fragments of about 5,000 and 850 bp. The 5,000 bp fragment was isolated and ligated to the 850 bp fragment from pDH-m4-TA/QA. The ligation was used to transform MC1000 competent cells. Vector DNA is isolated and analyzed by restriction and sequence analysis. Since the pURA-BStm4-181/9 vector after digestion with the above enzymes still retains the serine substitution at position 189, the final vector contains the changes of 99ser, 157arg, 167arg, 171arg, 180arg, 181ser and 189ser. The vector was designated pURA-m4-TS/QA and transformed into the BST-1C strain (PCT patent application PCT/US 88/00328) for fermentation evaluation. With arginine residues at positions 157, 167, 171, and 180, acetylation of the lysine residues is prevented and the concomitant occurrence of rbSt species with lower isoelectric points is avoided.

Similar methods can be used to replace the lysine residues at positions 157, 167, 171, and 180 with other acceptable amino acids, for example, glutamine. Similar methods can also be used to replace other lysines in other heterologous polypeptides which are subject to a similar acetylation process. Such acetylated molecules can be determined by the methods set forth herein.

**TABLE 1**

| LEVELS OF PI 8.2 AND 7.0 RBST SPECIES IN LOW YEAST EXTRACT FERMENTATION | | |
|---|---|---|
| Fermentation Sample¹ | Fractional Percent rbSt Species | |
| | pI 8.2 | pI 7.0 |
| 17 hrs | 71.4 | 28.6 |
| 19 hrs | 65.3 | 34.7 |
| 22 hrs | 72.9 | 27.1 |
| 26 hrs | 75.8 | 24.9 |
| 33 hrs | 75.0 | 25.0 |

| | | |
|---|---|---|
| ¹ Fermentation time in hours post-inoculation. Spontaneous induction of rbSt synthesis occurred at approximately 15 hours post-inoculation. | | |

CHART 1. Amino Acid Sequence of Bovine Somatotropin

10
ala phe pro ala met ser leu ser gly leu phe ala asn ala val

20 30
leu arg ala gln his leu his gln leu ala ala asp thr phe lys

40
glu phe glu arg thr tyr ile pro glu gly gln arg try ser ile

50 60
gln asn thr gln val ala phe cys phe ser glu thr ile pro ala

70
pro thr gly lys asn glu ala gln gln lys ser asp leu glu leu

80 90
leu arg ile ser leu leu leu ile gln ser trp leu gly pro leu

100
gln phe leu ser arg val phe thr asn ser leu val phe gly thr

110 120
ser asp arg val tyr glu lys leu lys asp leu glu glu gly ile

130
leu ala leu met arg glu leu glu asp gly thr pro arg ala gly

140 150
gln ile leu lys gln thr tyr asp lys phe asp thr asn met arg

160
ser asp asp ala leu leu lys asn try gly leu leu ser cys phe

170 180
arg lys asp leu his lys thr glu thr tyr leu arg val met lys

190
cys arg arg phe gly glu ala ser cys ala phe

## Claims

1. An animal somatotropin in which at least one lysine residue corresponding to residues 157, 167, 171 or 180 of the native somatotropin sequence shown in Chart 1 is replaced by arginine or glutamine.

2. The somatotropin of claim 1, which is mammalian.

3. The somatotropin of claim 2, which is bovine.

4. The somatotropin of claim 2, which is porcine.

5. The somatotropin of claim 3, wherein all of said lysine residues are replaced by arginine or glutamine.

6. The somatotropin of claim 5, wherein all of said lysine residues are replaced by arginine.

7. The somatotropin of claim 5, wherein all of said lysine residues are replaced by glutamine.

8. The somatotropin of claim 5, wherein lysine residues 157, 167 and 171 are replaced by arginine and lysine residue 180 is replaced by glutamine.

## Claims (Claims for the following Contracting State(s): ES)

1. A method for producing a modified animal somatotropin in which at least one lysine residue corresponding to residues 157, 167, 171 or 180 of the native somatotropin sequence shown in Chart 1 is replaced by arginine or glutamine.

2. The method of claim 1, wherein the somatotropin is mammalian.

3. The method of claim 2, wherein the somatotropin is bovine.

4. The method of claim 2, wherein the somatotropin is porcine.

5. The method of claim 3, wherein all of said lysine residues are replaced by arginine or glutamine.

6. The method of claim 5, wherein all of said lysine residues are replaced by arginine.

7. The method of claim 5, wherein all of said lysine residues are replaced by glutamine.

8. The method of claim 5, wherein lysine residues 157, 167 and 171 are replaced by arginine and lysine residue 180 is replaced by glutamine.
